# EUROPEAN PATENT SPECIFICATION

(11) **EP 0 546 094 B1**
(45) Date of publication and mention of the grant of the patent: **03.06.1998**
(21) Application number: 91917517.4
(22) Date of filing: 27.08.1991
(51) Int. Cl.: A61M 25/01

(54) **DUAL COIL GUIDEWIRE WITH RADIOPAQUE DISTAL TIP**
ZWEI SPIRALFEDERN FÜHRUNGSDRAHT MIT STRAHLUNDURCHLÄSSIGER DISTALER SPITZE
FIL DE GUIDAGE A DOUBLE BOBINE A EXTREMITE DISTALE RADIO-OPAQUE

(30) Priority: 29.08.1990 US 574629; 22.01.1991 US 644802
(43) Date of publication of application: 16.06.1993
(73) Proprietor: ADVANCED CARDIOVASCULAR SYSTEMS, INC., Santa Clara California 95052 (US)
(72) Inventor: HODGSON, William, S., Cohasset, MA 02025 (US); DHUWALIA, Jagdish, C., Irvine, CA 92715 (US); PFLUEGER, Russell, Newport Beach, CA 92659 (US)
(74) Representative: MacGregor, Gordon
(86) International application number: US9106132
(87) International publication number: WO9204072

(56) References cited:
- EP-A- 0 259 945
- WO-A-90/05486
- US-A- 3 749 086

## Description

### BACKGROUND OF THE INVENTION

The present invention is directed at the field of guidewires. Specifically, the present invention concerns a guidewire having a radiopaque tip.

Generally, guidewires include one or more coil springs fixed about a solid or tubular central core wire. Usually, the distal end of the guidewire is shapeable. This shapeability allows the surgeon to bend the distal end before insertion in the vascular system in conformance with the tortuous pathway of the desired vascular system segment through which the guidewire is being manipulated.

Guidewires are principally used for manipulating catheters through a patient's vessels, i.e. blood vessels. One specific application is the proper placement of a catheter in a patient's vascular system for a procedure known as percutaneous transluminal coronary angioplasty (PTCA).

A typical PTCA procedure involves percutaneously inserting a guiding catheter distal tip into the cardiovascular system of a patient and advanced therein until the distal tip thereof is in the coronary artery. A guidewire is introduced through the guiding catheter and advanced into the patient's coronary vasculature until the distal end of the guidewire crosses the lesion to be dilated. A dilatation catheter having an inflatable balloon on the distal portion thereof is advanced over the previously introduced guidewire, with the guidewire slidably disposed within an inner lumen of the dilatation catheter, until the dilatation balloon is properly positioned across the lesion. Once in position across the lesion, the balloon is inflated to a predetermined size with radiopaque liquid at a relatively high pressure to compress the atherosclerotic plaque of the lesion against the inside of the artery wall. The balloon is then deflated so that the dilatation catheter can be removed and blood flow resumed through the dilated artery.

Examples of guidewire designs are disclosed in WO-A-90/05486, EP-A-0259945, US-A-3,749,086, US-A 4,545,390, US-A-4,538,622, US-A-3,789,841, US-A-4,815,478, US-A-4,813,434, US-A-4,922,924, US-A-4,763,647, US-A-4,846,186 and US-A-4,886,067.

Numerous workers have devised guidewires to increase the steerability through the coronary system. For example, the tips are made more flexible by terminating the distal end of the core wire short of the distal end of the helical coil spring. A second inner helical coil spring is brazed at one end to the distal end of the core wire and at its opposite end to the distal end of the outer helical coil spring, see US-A-4,763,647, 4,886,067 and US-A-3,749,086.

Steering of the guidewire through the patient's coronary system is usually accomplished by viewing the guidewire via X-Ray. Visibility of the guidewire is achieved by forming at least a portion of the guidewire from a radiopaque material. This may be accomplished by many different ways. For example, a radiopaque spring is mounted to the end of the guidewire as disclosed in U.S. Patent 4,538,622. Other examples include forming the entire guidewire from a radiopaque spring.

While existing guidewires provide adequate steerability and radiopaqueness, further improvements are desirable.

### SUMMARY OF THE INVENTION

The present invention provides a guidewire according to claims 1 and 3, in which the precharacterising parts are based on US-A-4,763,647.

### DESCRIPTION OF THE DRAWINGS

The present invention may be better understood and the advantages will become apparent to those skilled in the art by reference to the accompanying drawings, wherein like reference numerals refer to like elements in the several figures, and wherein:
Figure 1 is a cross-sectional side view of a guidewire in accordance with an embodiment of the invention;

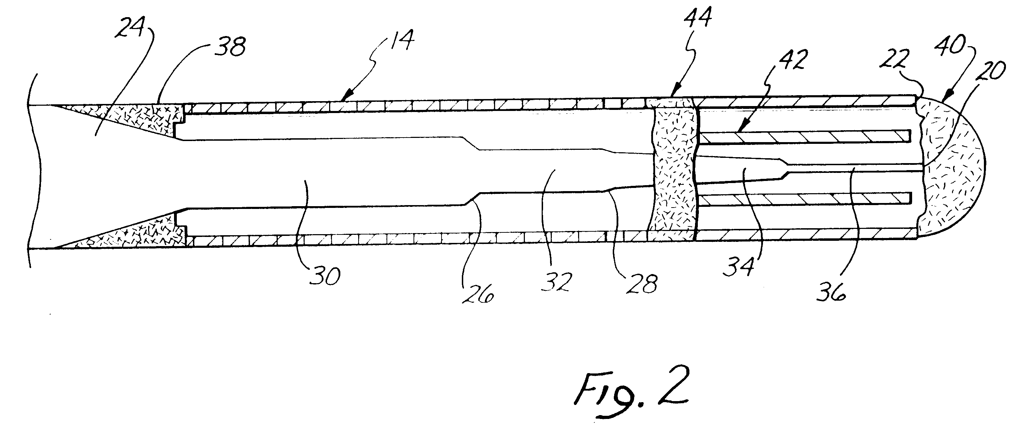

Figure 2 is a sectional, cross section of the distal end of the guidewire of Figure 1;
Figure 3 is a sectional cross section of a distal end of a guidewire in accordance with another embodiment of the invention;
Figure 4 is a further section cross section of a distal end of a guidewire in accordance with a still further embodiment of the invention; and
Figure 5 is a further embodiment of the invention.

### DESCRIPTION OF THE PREFERRED EMBODIMENTS

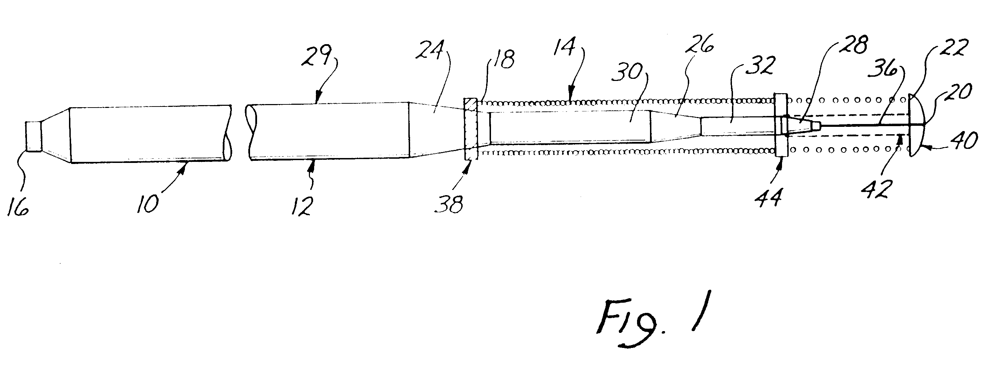

The invention is directed at a guidewire as seen generally in Figure 1 at 10. Guidewire 10 includes a centrally positioned core wire 12 and a outer helical coil spring 14. Centrally positioned core wire 12 and outer helical coil spring 14 have proximal ends 16 and 18, and distal ends 20 and 22, respectively. The distal ends 20 and 22 are commonly located, while the proximal end 18 is located distal of the proximal end 16.

Centrally positioned core wire 12 includes a main segment 29 from which extends multiple reduced diameter segments 30, 32 and 34. Proximally located and tapering to each of the reduced diameter segments 30, 32 and 34 are tapered portions 24, 26 and 28. At the very distal end of the centrally positioned core wire 12 is a tip portion 36. This tip portion 36 is of significantly smaller diameter in comparison to the distally located reduced diameter segment 34. This increases the flexibility of the tip portion 36. Preferably, tip portion 36 is flattened to provide for even greater flexibility. The outer diameters of the various segments of centrally positioned core wire 12, i.e.main segment 29 and reduced diameter segments 30, 32 and 34 is dependent upon the application of the finished guidewire 10. That is, the size of the vessel through which the guidewire 10 passes. This same rationale applies to the diameter or thickness for the tip portion 36.

The lengths of the main segment 29 and each reduced diameter segments 30, 32 and 34, is dependent upon the overall desired length for the guidewire 10. For example, main segment 29 may be 1.52 m(60"), with reduced diameter segments 30, 32 and 34 having lengths of 178 mm, 7.6 mm and 1.3 mm (7", 3" and 0.05"). The tip portion 36 could be 18 mm (0.7").

The outer helical coil spring 14 is brazed directly to centrally positioned core wire 12. Preferably the proximal end 18 of outer helical coil spring 14 is brazed to the first reduced diameter segment 30, as seen at 38, with the distal end 22 brazed to the distal end 20 of centrally positioned core wire 12, as seen at 40. Brazed end 40 is rounded to reduce potential damage to a patient's vessels as the guidewire 10 is manipulated therethrough.

A second radiopaque helical coil spring 42 is fitted in the outer helical coil spring 14. This second radiopaque helical coil spring 42 is positioned at the proximal end 22 and brazed directly to the centrally positioned core wire 12 and outer helical coil spring 14 at a proximal location as seen at 44. The brazing at 44 provides an additional safety joint. That is, some guidewires have the distal end of the core wire terminating before the distal end of the outer helical coil spring.

Breakage of the outer helical coil spring could result in leaving this broken portion of the helical coil spring in the patient upon withdrawal of the guidewire. Attempts to correct this deficiency, as disclosed in US-A-4,763,647 and US-A-4,886,067 include the brazing of the opposite ends of an inner disposed helical coil spring to the distal ends of both the core wire and the outer helical coil spring. This has been suggested as improving the distal tip flexibility. However, breakage of the outer helical coil spring may still result in loss of a portion of the spring due to the lesser strength of the inner helical coil spring. The guidewire of the preferred embodiment of the present invention overcomes this deficiency by providing for a secondary brazing 44 of the second radiopaque helical coil spring 42 to both the outer helical coil spring 14 and the centrally positioned core wire 12. The distal end of the second radiopaque helical coil spring 42 is not brazed to the brazed end 40.

The second radiopaque helical coil spring 42 is formed from a suitable material which does not appreciably reduce the overall flexibility of the guidewire 10 distal tip. For example, the second radiopaque helical coil spring 42 may be formed from Rhenium, Tungsten, Tantalum, Platinum or Gold. The outer helical coil spring 14 is typically formed from stainless steel.

The centrally positioned core wire 12 will have a decreasing flexibility from the first reduced diameter segment 30 through the last segment 34. The outer helical coil spring 14 will have the same degree of flexibility along its entire length. The resulting flexibility of the guidewire 10 increases in a direction from the proximal end to the distal end due to the increase in flexibility of the centrally positioned core wire 12 from its proximal to distal ends. The distal tip of the guidewire 10 has a greater flexibility than the remainder of the guidewire 10 due to the flattening of the tip portion 36. Preferably the second radiopaque helical coil spring 42 is formed with adjacent coils spaced apart to provide for greater flexibility. It should be noted that the second radiopaque helical coil spring 42 is only providing the radiopaque feature. This spring 42 need not provide any degree of stiffness to the guidewire 10 distal end so that the coils may be sufficiently spaced apart from adjacent coils to provide the distal end with a high degree of flexibility.

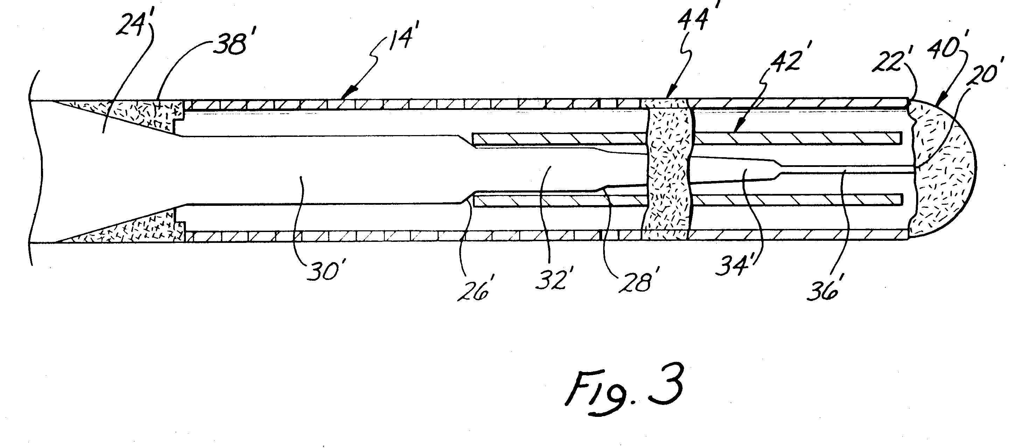

The overall length of the second radiopaque helical coil spring 42 is from about two to four centimeters. As seen in Figure 3, the second radiopaque helical coil spring 42' may in fact extend proximal the brazing 44. The remaining elements of the 10' are essentially the same as the already described guidewire 10, with like reference numeral indicating like elements.

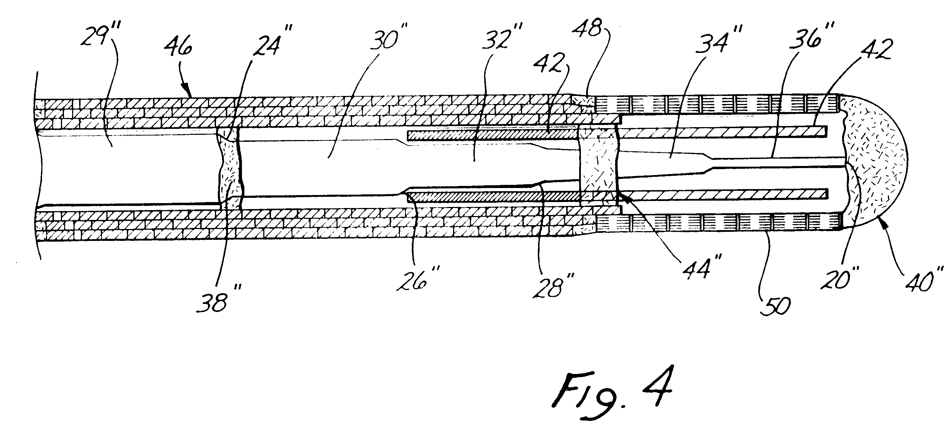

While the described embodiments provide for a single outer helical coil spring 14, other embodiments are contemplated. This embodiment, seen in Figure 4, replaces a portion of the outer helical coil spring 14 with a multiple helical coil spring arrangement, referred to as a trilayered spring 46. The trilayered spring 46 is formed by inserting one spring inside another, with this arrangement inserted into a third spring. This may be repeated to provide a multiple layered helical coil spring arrangement. One embodiment involves a three layered helical coil spring positioned one inside another as seen in Figure 4 as spring layers 52, 54 and 56. This spring arrangement is purchased from MicroSpring Company of Norwell, Massachusetts under the product name Triplex. A more detailed description of such a trilayered spring 46 is found in US-A-5052404.

The distal end of the trilayered spring 46, seen generally at 48 terminates proximal to the distal end 20'' of the centrally positioned core wire 12. This distal end 48 is typically positioned adjacent the brazing 44''. A separate helical coil spring 50 is brazed at one end to the trilayered spring 46 and at the opposite end to the distal end 20 of centrally positioned core wire 12. This helical coil spring 50 may be formed by extending one of the multiple springs to the distal end of the guidewire 10.

The remaining element of the guidewire 10' shown in Figure 4 are the same as those shown in the previous embodiments, with like reference numeral referring to like elements.

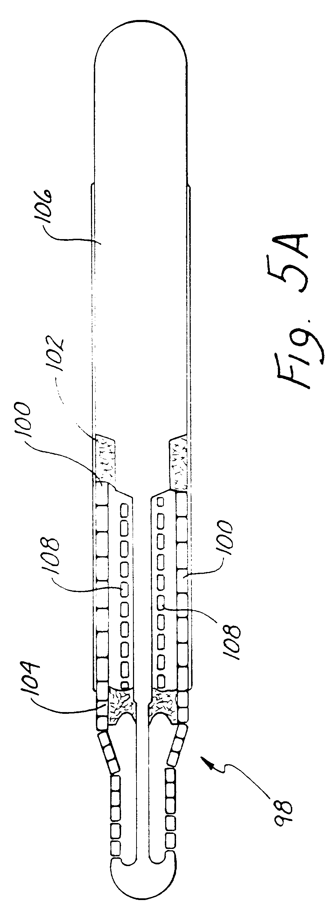

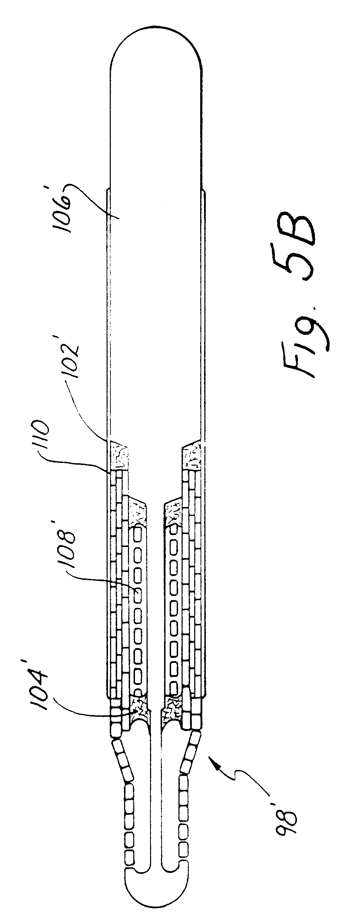

A still further embodiment of the invention is seen generally in Figure 5A, at 98. In this embodiment an outer helical coil spring 100 is brazed at locations 102 and 104 to the inner core wire 106. Placed between these two brazing locations 102 and 104, and between the helical spring 100 and the core wire 106 is radiopaque coil 108. This coil 108 may, or may not be brazed directly to the core wire 106, preferably, coil 108 is freely moveable between the two brazed locations 102 and 104.

Figure 5B is a modification of the guidewire embodiment illustrated in Figure 5A. The distinction is that the outer helical coil spring 100 is replaced with a trilayered spring assembly 110, similar to that described above for the embodiment of Figure 4. The remaining elements of the guidewire 98' are indicated with prime numbers similar to those used in Figure 5A.

## Claims

1. A guidewire comprising a core wire (12) having proximal and distal ends, a first helical coil spring (14) fit about the core wire and having a proximal end (18) secured to the core wire (12) at a location (38) distal to the proximal end (16) of the core wire and a distal end (22) secured to the distal end (20) of the core wire, and a second, radiopaque helical coil spring (42) having proximal and distal ends, the second helical coil spring (42) being fitted about the core wire and within the first helical coil spring (14), and being located at the distal end of the first helical coil spring (14),
characterised in that the second helical coil spring (42) has at least one unsecured end and is secured to the first helical coil spring (14) and the core wire (12) at a location proximal the distal ends of the core wire and the first helical coil spring (14).

2. The guidewire of Claim 1 wherein the proximal end of the second helical coil spring (42) is secured to the core wire (12).

3. A guidewire comprising a core wire (106) having proximal and distal ends, a first helical coil spring (100) fit about said core wire and having a proximal end secured to said core wire at a first location (102) distal the proximal end of said core wire, said first helical coil and said core wire being secured to each other at a second location (104) proximal of the distal ends of said first helical coil and said core wire, and a second radiopaque helical coil spring (108) having proximal and distal ends, said second helical coil spring being fitted about the core wire and within the first helical coil spring,
characterised in that said second coil spring is located at the proximal end of the first helical coil spring, the distal end of the second coil spring being secured to the first coil spring at said second location and the proximal end being unsecured.

4. The Guidewire of any preceding claim wherein said second helical coil spring (42,108) has unsecured ends.

5. The guidewire of any preceding claim wherein said second helical coil spring (42,108) is from two to four centimeters in length.

6. The guidewire of any preceding claim further including a rounded top portion at said distal ends (22,20) of said first helical coil spring (14,100) and core wire (12,106).

7. The guidewire of any preceding claim wherein said second helical coil spring is formed from Rhenium, Tungsten, Tantalum, Platinum or Gold.

8. The guidewire of any preceding claim where the first helical coil spring (46) includes a multilayered assembly with at least two helical springs (52,54,56) disposed one atop the other.

9. The guidewire of Claim 8 wherein the first helical spring coil includes a further helical spring (50) fit about said core wire and having a proximal end secured to the distal end of the multilayered assembly (52,54,56) and a distal end secured to the distal end (40') of the core wire.

## Patentansprüche

1. Führungsdraht, der folgendes aufweist:
einen Kerndraht (12), der ein proximales und ein distales Ende hat,
eine erste Schraubenfeder (14), die um den Kerndraht herum angebracht ist und ein proximales Ende (18), das an dem Kerndraht (12) an einer Stelle (38) befestigt ist, die in bezug auf das proximale Ende (16) des Kerndrahts distal ist, und ein distales Ende (22) hat, das an dem distalen Ende (20) des Kerndrahts befestigt ist,
und eine zweite, strahlenundurchlässige Schraubenfeder (42), die ein proximales und ein distales Ende hat, wobei die zweite Schraubenfeder (42) um den Kerndraht herum und innerhalb der ersten Schraubenfeder (14) angebracht und an dem distalen Ende der ersten Schraubenfeder (14) positioniert ist,
dadurch gekennzeichnet,
daß die zweite Schraubenfeder (42) wenigstens ein unbefestigtes Ende hat und an der ersten Schraubenfeder (14) und dem Kerndraht (12) an einer Stelle befestigt ist, die in bezug auf die distalen Enden des Kerndrahts und der ersten Schraubenfeder (14) proximal ist.

2. Führungsdraht nach Anspruch 1,
wobei das proximale Ende der zweiten Schraubenfeder (42) an dem Kerndraht (12) befestigt ist.

3. Führungsdraht, der folgendes aufweist:
einen Kerndraht (106), der ein proximales und ein distales Ende hat,
eine erste Schraubenfeder (100), die um den Kerndraht herum angebracht ist und ein proximales Ende hat, das an dem Kerndraht an einer ersten Stelle (102) befestigt ist,
die in bezug auf das proximale Ende des Kerndrahts distal ist, wobei die erste Schraubenfeder und der Kerndraht an einer zweiten Stelle (104) aneinander befestigt sind, die in bezug auf die distalen Enden der ersten Schraubenfeder und des Kerndrahts proximal ist,
und eine zweite, strahlenundurchlässige Schraubenfeder (108), die ein proximales und ein distales Ende hat, wobei die zweite Schraubenfeder um den Kerndraht herum und innerhalb der ersten Schraubenfeder angebracht ist,
dadurch gekennzeichnet,
daß die zweite Schraubenfeder an dem proximalen Ende der ersten Schraubenfeder positioniert ist, wobei das distale Ende der zweiten Schraubenfeder an der ersten Schraubenfeder an der zweiten Stelle befestigt ist und das proximale Ende unbefestigt ist.

4. Führungsdraht nach einem der vorhergehenden Ansprüche,
wobei die zweite Schraubenfeder (42, 108) unbefestigte Enden hat.

5. Führungsdraht nach einem der vorhergehenden Ansprüche,
wobei die zweite Schraubenfeder (42, 108) eine Länge von 2 bis 4 cm hat.

6. Führungsdraht nach einem der vorhergehenden Ansprüche,
der ferner einen gerundeten oberen Bereich an den distalen Enden (22, 20) der ersten Schraubenfeder (14, 100) und des Kerndrahts (12, 106) aufweist.

7. Führungsdraht nach einem der vorhergehenden Ansprüche,
wobei die zweite Schraubenfeder aus Rhenium, Wolfram, Tantal, Platin oder Gold besteht.

8. Führungsdraht nach einem der vorhergehenden Ansprüche,
wobei die erste Schraubenfeder (46) eine vielschichtige Anordnung aufweist, bei der wenigstens zwei Schraubenfedern (52, 54, 56) aufeinander angeordnet sind.

9. Führungsdraht nach Anspruch 8,
wobei die erste Schraubenfeder eine weitere Schraubenfeder (50) aufweist, die um den Kerndraht herum angebracht ist und ein proximales Ende, das an dem distalen Ende der vielschichtigen Anordnung (52, 54, 56) befestigt ist, und ein distales Ende hat, das an dem distalen Ende (40') des Kerndrahts befestigt ist.

## Revendications

1. Fil de guidage comprenant un fil d'âme (12) ayant une extrémité proximale et une extrémité distale, un premier ressort à enroulement hélicoïdal (14) disposé autour du fil d'âme et ayant une extrémité proximale (18) fixée au fil d'âme (12) à un endroit (38) distal par rapport à l'extrémité proximale (16) du fil d'âme et une extrémité distale (22) fixée à l'extrémité distale (20) du fil d'âme, et un deuxième ressort à enroulement hélicoïdal radio-opaque (42) ayant une extrémité proximale et une extrémité distale, le deuxième ressort à enroulement hélicoïdal (42) étant disposé autour du fil d'âme et à l'intérieur du premier ressort à enroulement hélicoïdal (14) et étant situé à l'extrémité distale du premier ressort à enroulement hélicoïdal (14),
caractérisé en ce que le deuxième ressort à enroulement hélicoïdal (42) présente au moins une extrémité non fixée, et il est fixé au premier ressort à enroulement hélicoïdal (14) et au fil d'âme (12) à un endroit proximal par rapport aux extrémités distales du fil d'âme et du premier ressort à enroulement hélicoïdal (14).

2. Fil de guidage suivant la revendication 1, dans lequel l'extrémité proximale du deuxième ressort à enroulement hélicoïdal (42) est fixée au fil d'âme (12).

3. Fil de guidage comprenant un fil d'âme (106) ayant une extrémité proximale et une extrémité distale, un premier ressort à enroulement hélicoïdal (100) disposé autour dudit fil d'âme et ayant une extrémité proximale fixée audit fil d'âme à un premier endroit (102) distal par rapport à l'extrémité proximale du dit fil d'âme, ledit premier ressort à enroulement hélicoïdal et ledit fil d'âme étant fixés l'un à l'autre à un deuxième endroit (104) proximal par rapport aux extrémités distales dudit premier ressort à enroulement hélicoïdal et dudit fil d'âme, et un deuxième ressort à enroulement hélicoïdal radio-opaque (108) ayant une extrémité proximale et une extrémité distale, ledit deuxième ressort à enroulement hélicoïdal étant disposé autour du fil d'âme et à l'intérieur du premier ressort à enroulement hélicoïdal,
caractérisé en ce que ledit deuxième ressort à enroulement hélicoïdal est placé à l'extrémité proximale du premier ressort à enroulement hélicoïdal, l'extrémité distale du deuxième ressort à enroulement hélicoïdal étant fixée au premier ressort à enroulement hélicoïdal audit deuxième endroit, et l'extrémité proximale étant non fixée.

4. Fil de guidage suivant une quelconque des revendications précédentes, dans lequel ledit deuxième ressort à enroulement hélicoïdal (42,108) a des extrémités non fixées.

5. Fil de guidage suivant une quelconque des revendications précédentes, dans lequel ledit deuxième ressort à enroulement hélicoïdal (42,108) a une longueur de 2 à 4 cm.

6. Fil de guidage suivant une quelconque des revendications précédentes, comprenant en outre un embout arrondi auxdites extrémités distales (22,20) dudit premier ressort à enroulement hélicoïdal (14,100) et du dit fil d'âme (12,106).

7. Fil de guidage suivant une quelconque des revendications précédentes, dans lequel ledit deuxième ressort à enroulement hélicoïdal est en Rhénium, Tungstène, Tantale, Platine ou Or.

8. Fil de guidage suivant une quelconque des revendications précédentes, dans lequel le premier ressort à enroulement hélicoïdal (46) comprend un assemblage multicouche comportant au moins deux ressorts hélicoïdaux (52,54,56) disposés l'un sur l'autre.

9. Fil de guidage suivant la revendication 8, dans lequel le premier ressort à enroulement hélicoïdal comprend un autre ressort hélicoïdal (50) disposé autour dudit fil d'âme et ayant une extrémité proximale fixée à l'extrémité distale de l'assemblage multicouche (52,54,56) et une extrémité distale fixée à l'extrémité distale (40') du fil d'âme.
